# EUROPEAN PATENT SPECIFICATION

(11) **EP 1 584 370 B1**
(45) Date of publication and mention of the grant of the patent: **03.09.2014**
(21) Application number: 05005224.0
(22) Date of filing: 10.03.2005
(51) Int. Cl.: C08L 3/04, C08L 3/06, C08L 3/08, A61K 8/06, A61K 8/73, B01F 17/00

(54) **Emulsifier**
Emulgator
Émulsifiant

(30) Priority: 22.03.2004 US 805857
(43) Date of publication of application: 12.10.2005
(73) Proprietor: Akzo Nobel N.V., 6824 BM Arnhem (NL)
(72) Inventor: Bonnardel, Valerie, 92700 Colombe (FR); Catterson, Florence, 8914 Aeugst-am-Albis (CH); Gestmann, Anja, 5702 Niederlenz (CH)
(74) Representative: Akzo Nobel IP Department

(56) References cited:
- EP-A- 0 332 027
- EP-A- 0 689 829
- EP-A- 0 913 406
- EP-A- 1 389 459
- WO-A-98/01109
- DATABASE CHEMABS CHEMICAL ABSTRACTS SERVICE, COLUMBUS, OHIO, US; "Oil-in-water emulsion cosmetics" XP002344845 retrieved from STN Database accession no. 128:26747 & JP 09 278644 A (NOEVIR K. K.) 28 October 1997 (1997-10-28)

## Description

The present invention relates to a novel emulsifier. In particular, the present invention is directed towards an emulsifier having at least one pregelatinized, crosslinked starch selected from a C₂-C₅ hydroxyalkyl starch and a C₂-C₁₈ acyl starch and at least one starch derivative containing a hydrophobic group or both a hydrophilic group and a hydrophobic group.

Emulsions are widely produced and used in industry as substances for consumption, or for application to surfaces as carriers of agents which are insoluble in water. Emulsions are found in cosmetics (lotions, creams, ointments), in cooking (sauces, creams), in pharmaceuticals (ointments, creams), in painting (low-odor paint), in the road industry (emulsified bitumen), in agrochemicals (plant protecting agents), in detergents, in rolling, iron and steel production and in the manufacture of various deposits or coatings (printing, adhesives, etc.). In cosmetics and pharmaceuticals, emulsions provide an effective means of an achieving a harmonious combination of ingredients of different types and properties in a uniform presentation easy to use in producing hygiene or beauty products.

Modified starches useful for emulsifying industrial products containing flavor oils are known. Such starches can be prepared by enzymatic degradation of the 1,4-α-D-glucosidic linkages from the non-reducing ends of a starch molecule, preferably employing β-amylase. This degradation can be carried out before or after the preparation of a starch derivative containing a hydrophobic group, or both hydrophilic and hydrophobic substituent groups.

EP 0 332 027 discloses such a starch emulsifier.

WO 98/01109 discloses cleaning and cosmetic preparations containing a pregelatinized, crosslinked starch selected from a C2-C5 hydroxyalkyl starch and a C2-C18-acyl-starch.

Emulsifiers useful in personal care or cosmetic compositions include polyose/fatty acid complex products formed from the reaction product of at least one polyose with at least one fatty acid, particularly in the form of a halide or the anhydride. The fatty acid can be saturated or unsaturated and can comprise from 8 to 28 carbon atoms.

Other emulsifiers useful in cosmetic or skin care compositions include aminomulticarboxylate starch derivatives.

Still there is a need for emulsifiers that are salt tolerant, as well as stable at a variety of temperature and processing conditions.

The present invention provides an emulsifier formed from a blend or mixture of at least a first starch component and a second starch component. The first starch component includes at least one pregelatinized, crosslinked starch such as a C₂-C₅ hydroxyalkyl starch or a C₂-C₁₈ acyl starch. The second starch component includes at least one starch derivative having a hydrophobic group or both a hydrophilic group and a hydrophobic group. The starch derivative is degraded by reaction with an exo-enzyme capable of cleaving 1,4-α-D-glucosidic linkages from non-reducing ends of starch but incapable of cleaving 1,6-α-D-glucosidic linkages of starch.

In one embodiment the first starch component is crosslinked using phosphorylation or C₄-C₁₈ alkane or alkene dicarboxylic acids. In this respect, useful crosslinking agents include phosphorous oxychloride, phosphorous pentoxide, sodium trimetaphosphate or mixtures thereof. In one aspect, the first starch component is crosslinked with phosphorous oxychloride.

In another embodiment the first starch component is a pregelatinized, crosslinked, hydroxypropylated starch or a pregelatinized, crosslinked, acylated starch. In this respect, useful first starch components include pregelatinized hydroxypropyl di-starch phosphate. Other useful first starch components include pregelatinized acetylated di-starch adipate.

The starch derivative is degraded by reaction with an exo-enzyme capable of cleaving 1,4-α-D-glucosidic linkages from non-reducing ends of starch, but incapable of cleaving 1,6-α-D-glucosidic linkages of starch. Such emulsifier achieves stable compositions that are salt tolerant and do not exhibit tackiness. Further, the emulsifier is ethoxylate-free and may be used in a variety of compositions, including cosmetic compositions.

The second starch component can be derivatized by reaction with an alkenyl cyclic dicarboxylic acid anhydride. Examples of alkenyl cyclic dicarboxylic acid anhydride suitable for use include octenyl succinic acid anhydride, dodecenyl succinic acid anhydride or mixtures thereof.

Examples of enzymes suitable in degrading the second starch component include β-amylase, exo-α-1,4-glucosidase, exo-1,4-α-D-glucan maltotetrahydrolase, exo-1,4-α-D-glucan maltohexahydrolase or combinations thereof. In one embodiment, the enzyme is β-amylase.

An example of the second starch component can be a converted octenyl succinic anhydride starch derivative degraded using β-amylase. The second starch component can be converted to a water fluidity of up to about 60.

In one embodiment, the emulsifier includes a pregelatinized hydroxypropyl di-starch phosphate and a converted octenyl succinic anhydride starch derivative degraded using β-amylase.

Either component of the emulsifier can be prepared from waxy corn starch. Further, the ratio of the first starch component to the second starch component can be 2:1.

In addition to the first and second starch components, the emulsifier can include various salts. The emulsifier can include up to 10% by weight of the salt.

The emulsifier is useful in cosmetic or personal care compositions. Stable cosmetic or personal care emulsion can be prepared by adding an emulsifying effective amount of the emulsifier to the composition.

As previously noted, the present invention is directed towards a novel emulsifier. The emulsifier includes at least two components prepared from base starches. Base starch, as used herein, includes all starches derived from any native source, any of which may be suitable for use herein. A native starch, as used herein, is one as it is found in nature. Also suitable are starches derived from a plant obtained by standard breeding techniques including crossbreeding, translocation, inversion, transformation or any other method of gene or chromosome engineering to include variations thereof. In addition, starch derived from a plant grown from artificial mutations and variations of the above generic starch, which may be produced by known standard methods of mutation breeding, are also suitable herein.

Typical sources for the starches are cereals, tubers, roots, legumes and fruits. The native source can be waxy varieties of corn (maize), pea, potato, sweet potato, banana, barley, wheat, rice, oat, sago, amaranth, tapioca (cassava), arrowroot, canna, and sorghum, as well as low and high amylose varieties thereof. As used herein, the term "low amylose" starch is intended to include a starch containing no more than about 10%, particularly no more than 5%, more particularly no more than 2% amylose by weight. As used herein, the term "high amylose" starch is intended to include a starch containing at least about 50%, more particularly at least about 70%, more particularly at least about 80% amylose by weight.

The first component comprises at least one pregelatinized, crosslinked starch selected from a C₂-C₅ hydroxyalkyl starch and a C₂-C₁₈ acyl starch. The term pregelatinized is intended to mean swollen starch particles that have lost their birefringence and/or Maltese crosses in polarized light. Such pregelatinized starches derivatives are substantially soluble in cold water without cooking. In this context "soluble" does not necessarily mean the formation of a true molecular solution, but may also mean a colloidal dispersion. In one embodiment, the starch is completely pregelatinized. Pregelatinization and techniques for achieving pregelatinization are known in the art and disclosed for example in U.S. Patent Nos. 4,465,702, 5,037,929, 5,131,953, and 5,149,799. Also see, Chapter XXII- "Production and Use of Pregelatinized Starch", Starch: Chemistry and Technology, Vol. III- Industrial Aspects, R.L. Whistler and E.F. Paschall, Editors, Academic Press, New York 1967.

Pregelatinization can be achieved by methods that include, without limitation, drum drying, extrusion and spray drying. In one embodiment, extrusion is used for the simultaneous cooking and drying of the starch (see, for example, U.S. Patent No. 3,137,592). This process makes use of the physical processing of a starch/water mixture at elevated temperatures and pressures which brings about the gelatinization of the starch, followed by expansion after leaving the nozzle with sudden evaporation of the water.

In one embodiment, pregelatinization is complete, thereby providing good solubility and eliminating undissolved particles that can give rise to an unpleasant, sandy feel in the composition.

In one embodiment, a majority of the starch granules are intact. Aqueous dispersions of pregelatinized starch derivatives having a largely intact granular structure typically have a more uniform smooth texture than aqueous dispersions of starches without a granular structure, which may have a slightly gritty feel. In the case of pregelatinized starches with an intact granular structure, the native internal structure of the hydrogen bonds is destroyed, but the external shape or form is maintained.

Another embodiment of this invention uses the process described in U.S. Patent No. 4,280,851 to produce the pregelatinized starch. An apparatus adapted for carrying out the process is described in U.S. Patent No. 4,600,472. In this process a mixture of the granular starch or starch derivative is cooked or gelatinized in the atomized state. The starch to be cooked is atomized through an atomizing opening into a nozzle arrangement in order to form a relatively finely divided sprayed material. In addition, a heating medium is injected through an opening in the nozzle arrangement into the sprayed material so as to heat the starch to the temperature necessary for gelatinization. A closed chamber surrounds the injection openings for the atomizing and heating medium and defines a ventilation opening positioned in such a way that the heated starch spray material can leave the chamber. The arrangement is such that during the passage of the starch spray material through the chamber, *i.e.,* from the atomizing opening to the ventilation opening, the time elapsed defines the starch's gelatinization time. The resulting spray dried, pregelatinized starch includes uniformly gelatinized starch granules. The granules are in the form of indented spheres, and are mostly whole, unbroken and swollen after hydration. Nozzles usable for producing such starches are also described in U.S. Patent No. 4,610,760.

For the production of suitable pregelatinized starches it is also possible to use the process of U.S. Patent No. 5,149,799. In this process starch is uniformly atomized and cooked by means of a single atomization stage in the presence of an aqueous medium. The atomization stage is performed in an apparatus having an internal mix, two-fluid spray drying nozzle, and it is coupled to a device for drying the cooked, atomized starch.

Spray dried, pregelatinized starches with suitable characteristics can also be produced by a continuous, coupled jet-cooking and spray-drying process. In one embodiment of this process, a starch suspension is gelatinized at 138°C to 160°C in a jet cooker with direct steam injection. The streams of starch suspension and steam are mixed in a cooking or boiling chamber. The outlet of the latter is connected to a pneumatic spray nozzle or a high pressure nozzle, which is located in a conventional spray dryer. The jet-cooked starch is directed at elevated temperature and pressure into the spray nozzle and can be atomized with cold air, hot air or steam. After atomizing, the hot, jet-cooked starch solution is handled in the same way as conventional spray dried starches. The drying process is adequately fast to prevent the starch molecules from retrograding during cooling and drying of the droplets. The spray dried starch is an amorphous material (*i.e*., it is substantially non-crystalline) that is easily soluble in water or colloidally dispersible. The dried product can also be agglomerated.

The first component is also crosslinked. Crosslinking of starch chains can be achieved by suitable crosslinking agents, including bi-functional compounds. In one embodiment, the crosslinking method used is phosphorylation wherein starch is reacted with phosphorous oxychloride, phosphorous pentoxide, and/or sodium trimetaphosphate. Two starch chains are crosslinked by an anionic P-O group. The anionic character of the crosslinking sites assists the emulsion-stabilizing action of the starch to be used according to the invention.

In another embodiment, the crosslinking method is by means of C₄-C₁₈ alkane or alkene dicarboxylic acids, which include without limitation C₄-C₈ alkane dicarboxylic acids such as adipic acid. The alkane or alkene dicarboxylic acid links two starch chains via ester bonds. It can be in straight or branched chain form. The derivatives may be obtained, for example, by reacting starch with the mixed anhydrides of dicarboxylic acid and acetic acid.

In one embodiment, less than 0.1 weight percent based on the dry starch crosslinking agent is used. In another embodiment, about 0.06 to 0.1 weight percent based on the dry starch crosslinking agent is used.

The first component is further modified to either a C₃-C₅ hydroxyalkyl.starch or a C₂-C₁₈ acyl starch. Such modifications and the techniques to achieve them are well known in the art.

In one embodiment, the first starch constituent is a C₃-C₅ hydroxyalkyl starch. The position of the hydroxyl group, which is bound to the starch backbone via an alkyl group with 3 to 5 carbon atoms in the alkyl group, is not critical and can be in the alpha (α) to omega (ω) position. In one suitable embodiment, the degree of substitution of hydroxyalkylation is about 0.08 to 0.3. The degree of substitution is the average number of substituted OH groups of the starch molecule per anhydroglucose unit. Hydroxyalkylation of starch can be brought about by reacting native starch with alkylene oxides having the appropriate number of carbon atoms, including without limitation hydroxypropylation by reaction of starch with propylene oxide. Starch useful according to the invention can also contain more than one hydroxyl group per alkyl group.

In another embodiment, the first starch constituent is a C₂-C₁₈ acyl starch. This starch can be formed by crosslinking the starch with C₄-C₁₈ alkanoate or alkenoate. The starch can be additionally acylated with a view to a suitable hydrophilic-lipophilic balance with a degree of substitution in one embodiment of 0 to 0.8 and in another of 0 to 0.5. Acylation may be achieved by reaction with acid anhydrides of the general formula (R-C(O))₂O, in which R is an alkyl group, such as methyl or ethyl, with succinic or maleic anhydride or their alkylated derivatives.

Suitable embodiments for the first component include without limitation hydroxypropyl di-starch phosphate and acetylated di-starch adipate. Also suitable are such starches derived from low amylose corn starch.

Processes use to prepare the first component can be conducted in any manner. However, one skilled in the art would understand the advantages of following certain steps. For example, hydroxypropylation is typically conducted before crosslinking with phosphorous oxychloride, as the hydroxypropylation process would destroy at least some of the crosslinking achieved if the crosslinking step was performed first.

The second component includes at least one starch derivative having a hydrophobic group or both a hydrophilic group and a hydrophobic group. This starch derivative is degraded by reaction with an exo-enzyme capable of cleaving 1,4-α-D-glucosidic linkages from non-reducing ends of starch, but incapable of cleaving 1,6-α-D-glucosidic linkages of starch.

In one embodiment, the starch of the second component is a pregelatinized starch derivative. Pregelatinization is achieved by the methods known in the art and described *supra.*

The starch of the second component can also be converted. Converted starches include, without limitation, fluidity or thin-boiling starches prepared by oxidation, enzyme conversion such as by α-amylase, mild acid hydrolysis, heat dextrinization, or mannox (manganese catalyzed degradation of starch as taught, for example, in U.S. Patent, No. 4,838,944). Such methods are well known in the art. (See, *e.g*.,⁻M. W. Rutenberg, "Starch and Its Modifications" in Handbook of Water-Soluble Gums and Resins, R. L. Davidson, editor, McGraw Hill, Inc., New York, N.Y., 1980, pp. 22-36) and/or U.S. Patent No. 4,035,235. A combination of these conversion techniques can also be used.

The second component starch can be derivatized by treatment with at least one reagent containing a hydrophobic moiety, and can additionally contain a hydrophilic moiety. The hydrophobic moiety can be an alkyl, alkenyl, aralkyl or aralkenyl group having at least five carbon atoms and, in one embodiment, five to twenty-four carbon atoms. In one embodiment, the hydrophilic moiety can be contributed by the reagent, or, in another embodiment, the starch's own hydroxyl groups can serve as the hydrophilic moiety and the reagent contributes only the hydrophobic moiety.

In one embodiment the starch is derivatized by reaction with an alkenyl cyclic dicarboxylic acid anhydride according to the method taught in U.S. Patent No. 2,661,349. However, any process for derivatizing starch that yields the desired blend of hydrophobic and hydrophilic functionality on the starch molecule can be used to derivatize the starch of the second component.

One suitable derivatization of the present invention is an octenyl succinate half ester derivative of a low amylose starch converted to water fluidity (WF) of up to about 60. Water Fluidity is an empirical test of viscosity measured on a scale of 0-90 wherein fluidity is the reciprocal of viscosity. Water Fluidity of starches is typically measured using a Thomas Rotational Shear-Type Viscometer (manufactured by Arthur H. Thomas Co., Philadelphia, Pa. 19106), standardized at 30°C with a standard oil having a viscosity of 24.73 cps., which oil requires 23.12+/-0.0.05 sec. for 100 revolutions. Accurate and reproducible measurements of the Water Fluidity are obtained by determining the time which elapses for 100 revolutions at different solids levels depending on the starch's degree of conversion (as conversion increases, the viscosity decreases).

The converted starch can be treated with at least 0.25% octenyl succinic acid anhydride (OSA). In another embodiment the starch is treated with at least 3.0% OSA, and in still another embodiment with at least 3.0% of dodecenyl succinic acid anhydride. In a further embodiment, the converted starch is further modified by hydroxyalkylation, as described *supra.*

The starch of the second component is further treated with an exo-enzyme. As used herein, exo-enzyme refers to an enzyme capable of cleaving the 1,4-alpha-D-glucosidic linkages from the non-reducing ends of starch, but incapable of cleaving 1,6-alpha-D-glucosidic linkages of starch. Non-limiting examples of exo-enzymes include β-amylase, exo-α-1,4-glucosidase, exo-1,4-α-D-glucan maltotetrahydrolase, and exo-1,4-α-D-glucan maltohexahydrolase.

Optimum parameters for enzyme activity can vary depending upon the enzyme used. Thus, the rate of enzyme degradation depends upon factors such as the type and concentration of enzyme used, the type and concentration of starch used, pH, temperature, the presence or absence of inhibitors, as well as other factors. Depending on the type of enzyme, or its source, various parameters can require adjustment in order to achieve the desired digestion rate. In one embodiment, the enzyme digestion reaction is carried out at the highest solids content feasible for facilitating subsequent drying of the starch while maintaining optimum reaction rates.

The process of this invention can use an enzyme in solution, an enzyme immobilized on a solid support, or any other methods of enzyme conversion known in the art.

At least one buffer can be used to ensure that pH will be in the optimum or desired range throughout degradation. Buffers include, without limitation, acetates, citrates and the salts of other weak acids. Other agents can also be used to optimize enzyme activity. The reaction can be carried out in any pH and temperature range suitable for the enzyme and base starch being used.

Enzymatic degradation is permitted to continue until the desired level of degradation is reached. The extent of degradation can be measured by various methods, including, for example, measuring the concentration of reducing sugars, change in viscosity or change in molecular weight using techniques well known in the art.

In one embodiment, starch degradation is allowed to proceed to a degree of from 13 to 55% by weight. In another embodiment, degradation is allowed to progress until up to 70% by weight of the starch has been hydrolyzed. In yet another embodiment, degradation is allowed to proceed until degradation essentially ceases.

After the desired degree of starch degradation has been reached, the enzyme can be deactivated by heat or other methods known in the art. Alternatively, the enzyme is not deactivated.

In one embodiment, the second component can be hydrolyzed to a dextrose equivalent (DE) of at least about 2; in another embodiment, at least about 5; in a third embodiment, at least about 10. Dextrose equivalence, as used herein, refers to the reducing power of a starch hydrolyzate. Each starch molecule has one reducing end; therefore, DE is inversely related to molecular weight. The DE of anhydrous D-glucose is defined as 100 and the DE of unhydrolyzed starch is virtually zero. In one embodiment, the DE of the blend of the two components will be at least about 2; in another embodiment, at least about 5; in a third embodiment, at least about 10.

In another embodiment, sugars can be added to achieve a DE of at least about 2; in another embodiment, at least about 5; in a third embodiment, at least about 10. In yet another embodiment, the starch derivative of the second component is not treated with enzymes to achieve *in situ* sugar formation. Instead, sugars are added to achieve the same DE level. Sugars, as used herein, refers to mono- di, and oligo-saccharides of up to about 10 glucose units, particularly those of up to about 3 glucose units, such as glucose, fructose, galactose, maltose, isomaltose, sucrose, lactose, raffinose, stachyose, fructosylsucrose, and maltooligosaccharides, particularly glucose, fructose, and maltose, as well as maltodextrins with a dextrose equivalent of from about 2 to about 50, particularly from about 5 to about 15. The increased DE can provide several advantages such as achieving and maintaining consistently high load levels, low oil exposure, increased oxidation resistance and increased ease of processing into the end use composition (*e.g*., cosmetics).

The practitioner will recognize that the sequence of steps in the process of this invention can be carried out in any order, though certain orders may present certain advantages.

The first and/or second components can be filtered through a filter aid such as granular or fibrous solids capable of forming a highly permeable filter cake in which very fine solids or slimy, deformable flocs can be trapped. Two common commercial filter aids are diatomaceous silica (also called diatomite or diatomaceous earth) and cellulosic fibers.

If purification is desired, the first and/or second components can be further purified by methods known in the art, such as dialysis, filtration, centrifugation or any other method known in the art.

The pH of the first and/or second component can be adjusted by methods known in the art, for example, by adding suitable pH-regulators such as citric acid, lactic acid, phosphoric acid, hydrochloric acid, sodium hydroxide, potassium hydroxide or triethanol amine. When using starch esters, a strong acid pH-value is typically avoided to prevent hydrolysis of the ester bonds. In one embodiment, the emulsifier has a pH value between about 2.5 and 12; in another between about 4 and 9.

The first and/or second components can also be dried or otherwise isolated using techniques known in the art, including without limitation spray-drying, drum-drying and freeze-drying. The components can be dried together or individually. In the alternative, the component(s) can be used in liquid or concentrated form. The emulsifier according to the invention can be provided in either an aqueous form or as a dry powder that is reconstituted in an aqueous medium upon use. The dried component(s) can further be agglomerated.

The emulsifier can be prepared by mixing the two components together. The ratio of first component to second component can be adjusted to obtain the attributes desired. For example, if more thickening is desired then more first component can be added. If more emulsifying is desired, then more of the second component can be added. In one embodiment, the ratio of first component to second component is at least 1:1 and no more than about 4:1 by weight. In another embodiment, the ratio of first component to second component is at least 1:1 and no more than about 3:1 by weight.

In one embodiment, the emulsifier includes a blend of a pregelatinized, hydroxypropylated waxy maize starch phosphate and an enzyme converted, octenyl succinic acid anhydride treated waxy maize starch. In another embodiment, these starches are present in a ratio of 2:1.

The emulsifier can be used at any level necessary to achieve the composition characteristics desired. In one embodiment, the emulsifier is used at a level of from about 1 to 10% by weight of the composition. In another embodiment, the emulsifier is used at a level of from about 1 to 7% by weight of the composition.

The resultant emulsifier can be used in a variety of compositions, including without limitation cosmetic and personal care compositions, paints, foods and beverages, pharmaceuticals and nutraceuticals, and home and fabric care compositions. Cosmetic and personal care compositions include, without limitation, moisturizing lotions and creams, sprays, mousses, gels including for the face and body, moisturizing cleansers and soaps, anti-aging products including anti-wrinkle products, anti-acne products, skin-lighteners, nourishing creams and lotions, firming and toning products, shaving creams, deodorants, color cosmetics including foundations, makeups, and lipsticks, suncare products such as sunscreens, suntan lotions, and after-sun products, hair conditioners and cream rinses, and shampoos, hair styling products including hairsprays, gels, and mousses, personal care wipes, baby care products, and bath and shower products.

Food and beverage compositions include without limitation sports and performance beverages, liquid gel products, and breads and bread products. Such products will also include food and beverage products for animals other than humans such as, without limitation, chickens, pigs, cattle, dogs and cats. In one embodiment, the emulsifier may also provide other functionality to food and beverage compositions such as that of improvers and softening agents for bread and bread products which would enhance crumb structure, softness, shelf-life and/or volume.

The compositions can include other optional components commonly used in the industry, which can vary greatly depending upon the type of composition and the functionality and properties desired. For example, cosmetic and personal care applications can also include, without limitation, aesthetic modifiers, UV filters, humectants, moisturizers, emollients, solvents, chelating agents, vitamins, antioxidants, botanical extracts, pH adjusting and neutralizing agents, preservatives, fragrances, active ingredients (anti-aging agents, firming or toning agents, etc.), dyes and pigments, conditioning agents, chelating agents, opacifiers, and foaming or antifoaming agents. Other emulsifiers and/or surfactants can be added, but are not necessary for providing emulsification functionality.

In one embodiment, the compositions are substantially surfactant-free and the emulsifier of the present invention is the only emulsifier present in an emulsifying-effective amount. In another embodiment, no surfactant is added and no emulsifier is added to the composition other than the emulsifier of the invention.

The emulsifier emulsifies oil-in-water emulsions for use in such compositions by inclusion in the aqueous phase using techniques commonly used and known in the art. The emulsifier is substantially dissolved in the aqueous phase.

The hydrophobic phase can contain, without limitation, liquid or solid fatty acid triglycerides, fatty acid monoesters or diesters, silicones, long-chain alcohols, and/or vitamins. In one embodiment, the composition contains at least about 1% of a hydrophobic phase by weight of the composition; in another, 5 to 25% by weight; and in a third, up to 70 % by weight. The hydrophobic phase can be finely dispersed.

Emulsions include, without limitation, those in the form of a cream, lotion or milk or spray. As a function of the characteristics and quantity of the aqueous or hydrophobic phase, the emulsion-type compositions can contain different types of emulsions, e.g., binary oil-in-water or water-in-oil systems or multiple phase systems such as water-in-oil-in-water or oil-in-water-in-oil systems. In another embodiment, the emulsion is in the form of a high viscosity gel.

The compositions can be adjusted to have a variety of textures, which can range from oily to creamy to waxy. The emulsifiers provide characteristics desirable from a dermatological standpoint. For example, they increase water retention capacity of skin, as well as make it smooth and flexible. Cosmetics containing a starch derivative used according to the invention can spread very well onto skin and do not leave behind a sticky feeling.

The emulsifier can also thicken the aqueous phase of the emulsion to a desired consistency. In one embodiment, the emulsifier provides a viscosity to the aqueous phase of 400 to 30,000 cps (mPa).

The emulsifier is substantially ethoxylate-free. In one embodiment, the emulsifier contains less than 0.1 % ethoxylate by weight; in another less than 0.01% ethoxylate by weight; and in still another no ethoxylate based on the weight of the emulsifier. Further, a natural base is advantageous over synthetic bases in many respects, including labeling. When used in a cosmetic composition or other composition that can come into contact with a consumer's skin, low (or no) ethoxylate level and/or natural base reduces adverse effects on many consumers, including skin irritation and sensitization, compared to many commonly used emulsifiers. For example, polyethylene glycol (PEG) is known to enable transport of pollutants to and into skin.

The emulsifier of this invention has the further advantage of being salt tolerant. Many commonly used emulsifiers, particularly in the cosmetic and personal care industry, have poor or no salt tolerance, resulting in instability upon inclusions of salts. With the increased popularity of mineral salts, plant and sea extracts and vitamins, salt tolerance of ingredients has become more important. In one embodiment of this invention, the composition containing the emulsifier is stable with a salt content of up to 5% by weight, and in another with a salt content of up to 10% by weight. In another embodiment, the composition contains at least 1% salt by weight, and in another at least 5% salt by weight. Salt tolerance is, as is known in the art, dependent in part on the other ingredients used in the composition.

The emulsifier exhibits freeze-thaw stability and high temperature stability, making them useful in of compositions made under a variety of processing conditions. The emulsifier also has the processing advantage of not needing to be cooked (although it may be cooked) and thus may be added to a cold or hot process, with or without shear.

The emulsifier also provides a pleasant mouth- or skin-feel to the composition. This is done without significant tackiness, or grittiness, both viewed adversely by the consumer.

### EXAMPLES

The following examples are presented to further illustrate and explain the present invention and should not be taken as limiting in any regard. All percents used are on a weight/weight basis.

In the examples below, the following procedures/tests are used.

Funnel viscosity. To measure funnel viscosity, 38 g of the converted starch (anhydrous basis) was weighed into a tared 250 ml beaker (stainless steel) containing a thermometer and brought to 200 g total weight with distilled water. The sample was mixed to dissolve any lumps and heated or cooled to 22°C (72°F). A total of 100 ml of the cooked starch dispersion was measured into a graduated cylinder. It was then poured into a calibrated funnel while using a finger to close the orifice. A small amount was allowed to flow into the graduate to remove any trapped air, and the complete balance remaining in the graduate was poured back into the funnel. Using a timer, the time required for the 100 ml sample to flow through the apex of the funnel was recorded.

The funnel was a standard 58 degree, thick-wall, resistance glass funnel whose top diameter was about 9-10 cm with the inside diameter of the stem being about 0.381 cm. The funnel was calibrated so as to allow 100 ml of water to go through in 6 seconds using the above procedure.

Stability testing - The cosmetic preparations described below were stability tested under the following conditions: The cosmetic preparation is stored in 125 ml glass jars for 1 months at 45°C, 3 freeze/thaw cycles at -20°C, and 3 months at room temperature. Stability is achieved if no significant changes in viscosity, pH, texture, color or odor are observed.

Brookfield viscosity - To measure the viscosity of cosmetic preparations described below, a Brookfield DV-I Heliopath viscometer is equipped with spindle C (the use of another spindle is notified, when appropriate). The measurement is carried out at 20 rpm at room temperature in a 125 ml glass jar.

pH measurements - pH of cosmetic preparations described below is measured with a pH-meter Orion 410A equipped with an Orion Ag/AgCl Sure-Flow Electrode at room temperature.

In the Examples, the following designations and trade names are used.

**Table 1 - Designations and Trade Names**

| **Trade Name** | **Manufacturer** | **INCI Name** |
|---|---|---|
| Neo Heliopan AV | Symrise | Ethylhexyl Methoxycinnamate |
| Neo Heliopan OS | Symrise | Ethylhexyl Salicylate |
| Parsol 1789 | Roche | Butyl Methoxydibenzoylmethane |
| Pricerine 9091 | Uniqema | Glycerin |
| Carbopol 1382 | Noveon | Carbomer |
| DC 193 | Dow Coming | PEG-12 Dimethicone |
| DC 200 | Dow Corning | Dimethicone |
| Versene 100 | Dow Chemicals | Tetrasodium EDTA |
| Phenonip | Nipa | Phenoxyethanol & Methylparaben & Ethylparaben & Propylparaben |
| Triethanolamine | Merck/Rona | Triethanolamine |
| Sunflower Oil refined | Jan Dekker | Sunflower Oil |
| Lincol BAS | Eigenmann & Veronelli | C12-15 Alkylbenzoate |
| 1,2-Propandiol | Merck/Rona | Propylene Glycol |
| Dry-Flo AF | National Starch & Chemical | Corn Starch Modified |
| Structure XL | National Starch & Chemical | Hydroxypropyl Starch Phosphate |
| Locron LIC | Clariant | Aluminium Chlorohydrate |
| Arlacel 1690 | Uniqema | Sorbitan Isostearate & Polyglyceryl-3 Ricinoleate |
| Germaben IIE | International Specialty Polymers | Propylene Glycol & Diazolidinylurea & Methylparaben & Propylparaben |
| Arlamol E | Uniqema | PPG-15 stearyl Ether |
| Atlas G-2330 | Uniqema | Sorbeth-30 |
| Euxyl K400 | Schülke & Mayer | Phenoxyethanol & Methyldibromo Glyutaronitril |
| Estol 3603 | Uniqema | Caprylic/Capric Triglyceride |
| Estol 1514 | Uniqema | Isopropyl Myristate |
| Hansanol 1214 | Hansa Chemie | C12-C14 Fatty Alcohol |
| Drakeol 10 LT | Penreco | Mineral Oil |
| Estol 1517 | Uniqema | Isopropyl Palmitate |
| Pionier 7028 | Hansen & Rosenthal | Mineral Oil |
| Sodium Chloride p.A. | Fluka | Sodium Chloride |
| Calcium Chloride p.A. | Fluka | Calcium Chloride |
| Crodacol C95 | Croda | Cetyl Alcohol |
| Pristerene 4911 | Uniqema | Stearic Acid |
| Crodacol CS90 | Croda | Cetearyl Alcohol |
| Veegum Ultra | Vanderbilt Corp. | Magnesium Aluminium Silicate |
| Keltrol CG RD | Kelco | Xanthan Gum |

### EXAMPLE 1 - Preparation of Starch Component 1

### EXAMPLE 1A - Crosslinked Hydroxypropylated Waxy Corn

A total of 1000 parts waxy corn starch was introduced into a reaction vessel containing a solution of 18.75 parts sodium hydroxide and 250 parts sodium sulfate in 1500 parts water. 84 grams (8.4% on the weight of starch) of propylene oxide was added and the vessel was sealed. The contents were allowed to react for 16 hours at 40°C while the vessel was continuously tumbled to assure uniform suspension of the starch throughout the reaction time. After 16 hours, the vessel was removed from the tumbler, placed in a continuously agitated container and allowed to cool to 30°C.

At that point, 0.175 gram (0.0175% on starch weight or 0.106 mL) of phosphorous oxychloride was added and allowed to react for 30 minutes. The pH of the resultant suspension was then adjusted to 3.0-3.4 by the addition of a 25% sulfuric acid solution and held for 1 hour. The pH was finally adjusted to 5.5 with 3% sodium hydroxide, and the hydroxypropylated/crosslinked starch was recovered by filtration, washed two times with 1500 parts water. The starch was then cooked and dried using the methods of U.S. Patent Nos. 4,280,851 and 4,600,472.

### EXAMPLE 1B - STMP Crosslinking

A total of 1000 parts waxy corn starch was introduced into a reaction vessel containing a solution of 18.75 parts sodium hydroxide, 250 parts sodium sulfate and the desired amount of STMP in 1500 parts water. The required amount of propylene oxide was added and the vessel was sealed. The contents were allowed to react for 16 hours at 40°C while the vessel was continuously tumbled to assure uniform suspension of the starch throughout the reaction time. After 16 hours, the vessel was removed from the tumbler, placed in a continuously agitated container and allowed to cool to 30°C.

The pH of the resultant suspension was then adjusted to 3.0-3.4 by the addition of a 25% sulfuric acid solution and held for 1 hour. The pH was finally adjusted to 5.5 with 3% sodium hydroxide, and the hydroxypropylated/crosslinked starch was recovered by filtration, washed two times with 1500 parts water. The starch was then cooked and spray dried.

### EXAMPLE 1C - Adipic-Acetic Crosslinking

The adipic-acetic crosslinking reagent was prepared by adding 20 gm adipic acid to 180 gram acetic anhydride on a 500 ml Erlenmeyer flask placed in a water bath. While agitating the mixture, the water bath is heated to 90°C gradually over 1 hour and held at 90°C for an additional hour. The mixture is then cooled to room temperature and stored in a glass jar.

A total of 1000 parts waxy corn starch was introduced into a reaction vessel containing a solution of 18.75 parts sodium hydroxide and 250 parts sodium sulfate in 1500 parts water. The required amount of propylene oxide was added and the vessel was sealed. The contents were allowed to react for 16 hours at 40°C while the vessel was continuously tumbled to assure uniform suspension of the starch throughout the reaction time. After 16 hours, the vessel was removed from the tumbler, placed in a continuously agitated container and allowed to cool to 30°C.

The pH was adjusted to 8.0 (using 25% sulfuric acid) and the desired amount of adipic-acetic reagent was added while the pH was controlled to a range of 7.8 and 8.2 with a 3% sodium hydroxide solution. Once all adipic-acetic reagent was added and pH stabilized, the pH was then adjusted to 3.0-3.4 by the addition of a 25% sulfuric acid solution and held for 1 hour. The pH was finally adjusted to 5.5 with 3% sodium hydroxide, and the hydroxypropylated / crosslinked starch was recovered by filtration, washed two times with 1500 parts water, cooked, and dried.

### EXAMPLE 1D - Acetic anhydride with adipic-acetic crosslinking

A total of 1000 parts waxy corn starch was introduced into a reaction vessel containing 1500 parts water at 25°C. The pH was adjusted to 8.0 (using 3% sodium hydroxide) and the desired amount of both acetic anhydride and adipic-acetic reagent (prepared as above) was slowly added while the pH was controlled to a range of 7.8 and 8.2 with a 3% sodium hydroxide solution. Once all acetic anhydride and adipic-acetic reagents were added and the pH stabilized, the pH was then adjusted to 5.5 with dilute hydrochloric acid, and the acetylated / crosslinked starch was recovered by filtration, washed two times with 1500 parts water, cooked and dried.

### EXAMPLE 1E - Example 1A was repeated using tapioca starch in place of the corn starch.

### EXAMPLE 2 - Preparation of Starch Component 2

### EXAMPLE 2A - Preparation of OSA modified Waxy Maize

An octenyl succinate derivative (OSA) of waxy maize starch was prepared by the method disclosed in Example II of U.S. Pat. No. 2,661,349 except that the corn starch was replaced by waxy maize. In addition, the starch was reacted with 3% octenyl succinic acid anhydride rather than with 0.5% as disclosed in the reference. A 28% aqueous slurry of the OSA waxy maize was jet cooked at approximately 149°C (300°F). Thereafter, the cooked OSA waxy maize was placed in a constant temperature bath and maintained at 55°C with constant stirring. The pH was adjusted to 5.3 with 3% hydrochloric acid.

The cooked OSA waxy maize dispersion was divided into four batches and a different level of barley β-amylase (1,4-α-D-glucan maltohydrolase (E.C. 3.2.1.2), obtained from Fermco Biochemics, Inc., Elk Grove Village, Illinois) was added to each batch. The amounts of enzyme added were 168, 334, 840 and 1,110 DP degree per 100 g dry basis of OSA waxy maize for approximately eight hours.

The degree of degradation was determined by monitoring the funnel viscosity of the dispersion. Accordingly, the level of α-amylase contamination present in the barley β-amylase was monitored and limited to no more than 0.4 DU/ml of enzyme solution so that the viscosity was not affected by this variable. A dextrinizing unit (DU) is the quantity of α-amylase that will dextrinize soluble starch in the presence of excess β-amylase at the rate of 1 gm/hour at 20°C.

By carefully controlling the parameters of the funnel viscosity test and limiting α-amylase contamination, the degree of starch degradation by β-amylase was correlated to the loss in viscosity. The reducing sugars were measured by the Fehling method to confirm the degree of degradation.

In this example, the desired enzyme reaction end point was reached within a funnel viscosity range from 9-50 seconds. The reducing sugars content of these samples ranged from 29-35%. The corresponding degradation of the starch, by weight, ranged from 58-70%. When the targeted viscosity was reached, the enzyme was deactivated by injection of live steam into the reaction solution until a temperature of at least 75°C was attained and held for at least 15 min. The batches were then spray-dried at an inlet temperature of 200-210°C and an outlet temperature of 85-90°C using a standard #22 11/4 J nozzle obtained from Spraying Systems Company. The spray-dried starch product was screened through #40 mesh screen.

### EXAMPLE 2B - Removal of Maltose

β-amylase degraded OSA waxy maize prepared by the method set forth in Example 2A was subjected to dialysis to remove maltose. The starch was dispersed in distilled water at 15-20% solids and placed in a dialysis tube (obtained from Spectropor Membrane) that retained molecules with molecular weights in excess of 6,000-8,000. The dispersion was dialyzed against distilled water until the dialyzate was free of all organic materials. The starch was then collected by precipitation with ethanol.

### EXAMPLE 2C - Preparation of DSA modified Waxy Maize

- Example 2A was repeated except that dodecenyl succinic acid anhydride (DSA) was substituted for octenyl succinic acid anhydride.

### EXAMPLE 2D - Preparation of OSA modified Waxy Maize

Example 2A was repeated with a reversal in the sequence of steps. A 28% solids aqueous slurry of waxy maize starch was prepared, the pH was adjusted to 6.0-6.3 by addition of 3% NaOH. The slurry was jet-cooked at approximately 149°C (300°F). The cooked starch was placed in a constant temperature water bath and maintained at 55-60°C with constant stirring. The barley β-amylase used in Example 2A was added to the cooked starch at a concentration of 1,650 DP degree per 100 g dry basis of starch. The batch of β-amylase used in this example contained 0.9 DU/ml of α-amylase activity. The degree of degradation was monitored by the funnel viscosity procedure.

When the starch had reached a funnel viscosity of 30 seconds, the enzyme was deactivated by adding 10% HCl to lower the pH to 3.5-4.0 and holding the starch at this pH for 30-60 minutes. After deactivation, the pH was adjusted to 7.0 by adding 3% NaOH.

The OSA derivative was prepared by thoroughly blending 3 g of octenyl succinic acid anhydride per 100 g dry weight basis of starch into the neutralized, debranched starch dispersion. The reaction was permitted to continue at room temperature with agitation for 4 hours. A portion of this starch dispersion was spray-dried by the method set forth in Example 2A.

### EXAMPLE 2E - Preparation of OSA modified Waxy Maize Blend

500 grams of waxy maize starch were slurried in 750 ml water. The pH was adjusted to 7.5 using 3% sodium hydroxide. 15 grams of octenyl succinic anhydride (OSA) were added in one-third increments every thirty minutes while maintaining the pH at 7.5 using 3% sodium hydroxide and constant agitation. The starch was then filtered out and washed with 750 ml water. The starch was then reslurried in 500 ml water and the pH adjusted to 5.5 with 3:1 hydrochloric acid. The starch was then filtered, washed with 750 ml water, and air dried to produce an OSA starch. 600 grams of such OSA derivatized starch were mixed with 400 grams glucose.

### EXAMPLE 2F - Preparation of CWS OSA modified Waxy Maize Blend

A cold water soluble, mildly acid degraded OSA starch was prepared by using the derivatized starch of Example 2E, hydrolyzing using acid to a fluidity of about 60, and spray drying the starch. 600 grams of this starch was mixed with 400 grams of glucose.

### EXAMPLE 2G - Preparation of CWS OSA modified Waxy Maize Blend

500 grams of the starch prepared as in Example 2F were mixed with 500 grams maltose.

### EXAMPLE 2H - Preparation of OSA modified Waxy Maize Blend

340 grams of an OSA derivatized starch prepared as in Example 2E were mixed with 660 grams maltose.

### EXAMPLE 2I - Preparation of OSA modified Waxy Maize Blend

550 grams of an OSA derivatized starch prepared as in Example 2E were mixed with 450 grams glucose.

### EXAMPLE 2J - Preparation of OSA modified Waxy Maize Blend

500 grams of an OSA derivatized starch prepared as in Example 2E were mixed with 500 grams glucose.

### EXAMPLE 2K - Preparation of OSA modified Waxy Maize Blend

400 grams of an OSA derivatized starch prepared as in Example 2E were mixed with 600 grams glucose.

### EXAMPLE 3 -

The following samples of the emulsifier blend of this invention were made - Samples 4,5 and 6 are not representative of the invention.

**Table 2 - Emulsifiers**

| **Sample** | **Component 1** | **Component 2** |
|---|---|---|
| Sample 1 | 50% starch of Example 1A | 50% starch of Example 2A |
| Sample 2 | 67% starch of Example 1A | 33% starch of Example 2A |
| Sample 3 | 80% starch of Example 1A | 20% starch of Example 2A |
| Sample 4 | 50% starch of Example 1A | 50% starch of Example 2F |
| Sample 5 | 67% starch of Example 1A | 33% starch of Example 2F |
| Sample 6 | 80 % starch of Example 1A | 20% starch of Example 2F |

### Example 4 -

The following creams were prepared using the emulsifier described in Example 3 as Sample 1 - Sample 6 -

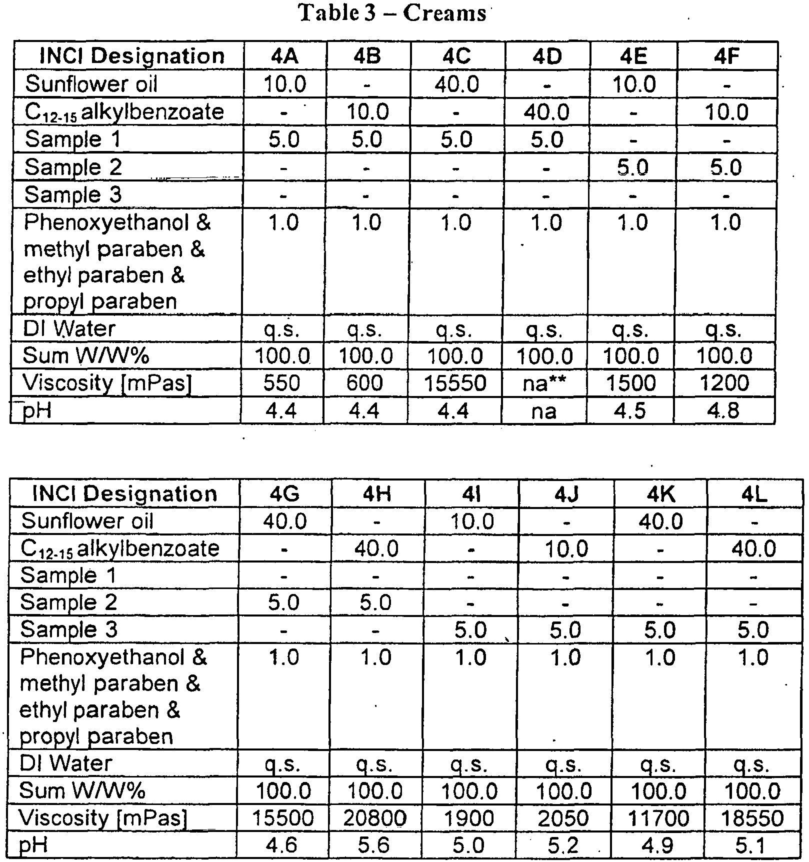

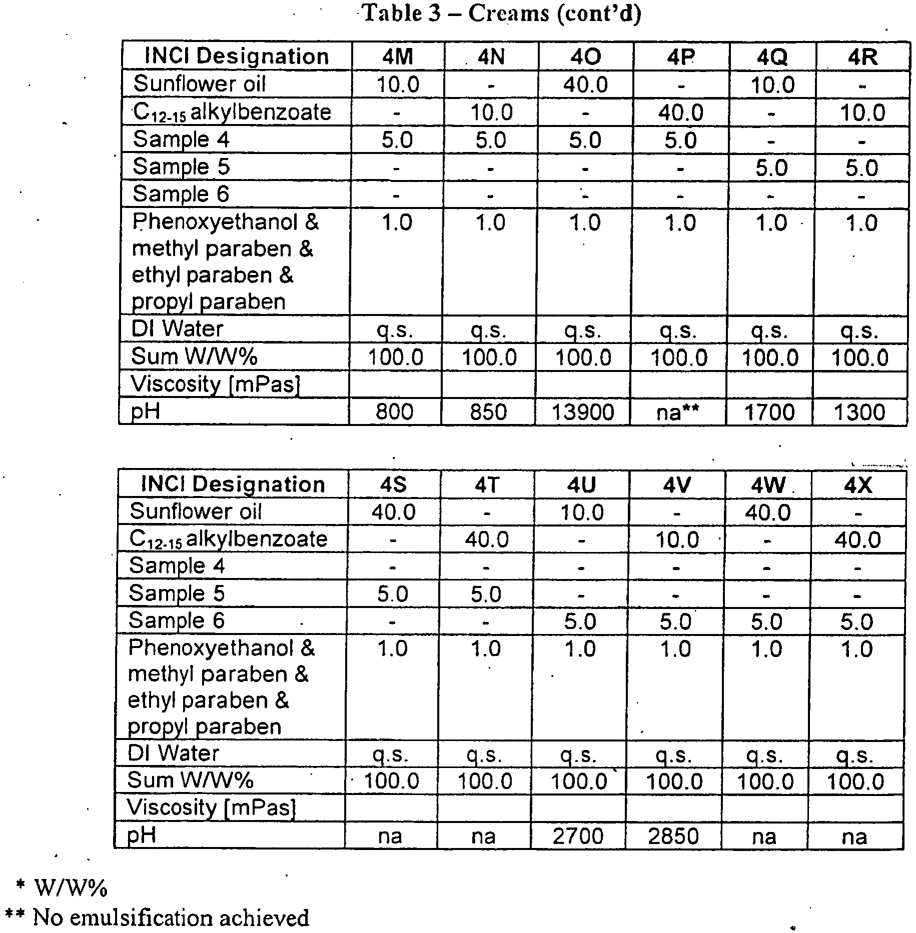

Procedural - The emulsifier was dispersed in deionized (DI) water and stirred until hydration was fully achieved. The preservative and oil were added while stirring using an overhead mixer at 900 rpm. The mixture was homogenized for one minute at 13,000 rpm.

### EXAMPLE 5 -

### EXAMPLE 5A - O/W Lotion -

**Table 4 - O/W Lotion**

| **INCI Designation** | **w/w%** |
|---|---|
| Mineral oil | 10.0 |
| Sample 2 | 5.0 |
| Isopropyl palmitate | 5.0 |
| Glycerin | 3.0 |
| Phenoxyethanol & methyl paraben & ethyl paraben & propyl paraben | 1.0 |
| DI Water | ad 100.0 |

Procedural - The emulsifier was dispersed in a mixture of DI water and glycerin and stirred until the starch was fully hydrated. The other components were premixed and added to the water phase while stirring at 900 rpm. The mixture was then homogenized for two minutes at 10,000·rpm.

| | |
|---|---|
| Viscosity | 5,000 mPas |
| pH | 5.0 |

### EXAMPLE 5B - O/W Milk -

**Table 5 - O/W Milk**

| **INCI Designation** | **W/W%** |
|---|---|
| Mineral oil | 8.0 |
| Sample 2 | 4.0 |
| PPG-15 stearyl ether | 3.5 |
| propylene glycol & diazolidinyl urea & methyl paraben & propyl paraben | 0.25 |
| DI Water | ad 100.0 |

Procedural - The emulsifier was dispersed in DI water and stirred until the starch was fully hydrated while heating to 80°C. The oil components were premixed, heated to 80°C and added to the water phase while stirring at 900 rpm. Stirring continued while cooling, and the preservative were added at 30°C.

| | |
|---|---|
| Viscosity | 2,000 mPas |
| pH | 5.3 |

### EXAMPLE 5C - Cream -

**Table 6 - Cream**

| **INCI Designation** | **W/W%** |
|---|---|
| Sample 2 | 5.0 |
| Corn starch modified | 4.0 |
| Propylene glycol | 4.0 |
| Ethylhexyl salicylate | 3.5 |
| Ethylhexyl methoxycinnamate | 3.1 |
| Glycerin | 2.0 |
| Hydroxypropyl starch phosphate | 1.0 |
| Phenoxyethanol & methyl paraben & ethyl paraben & propyl paraben | 1.0 |
| Tetrasodium EDTA | 0.2 |
| Carbomer | 0.15 |
| D.I. Water | ad 100.0 |
| Triethanolamine | ad pH 6.5 |

Procedural - Carbopol, emulsifier and hydroxypropyl starch phosphate were dispersed in D.I. water and stirred until all ingredients were fully hydrated. Tetrasodium EDTA and glycerin were added and heated to 80°C. The other components, with exception of triethanolamine, corn starch modified and propylene glycol, were premixed, heated to 80°C and added to the water phase while stirring at 900 rpm. Stirring continued while cooling to 45°C. Corn starch modified was dispersed in propylene glycol and added to the emulsion. The mixture was homogenized for two minutes at 10,000 rpm and pH was adjusted with triethanolamine.

| | |
|---|---|
| Viscosity | 19,500 mPas |
| pH | 6.5 |

### EXAMPLE 5D - AP/Deo Lotion

**Table 7 - Lotion**

| **INCI Designation** | **W/W%** |
|---|---|
| Sample 2 | 4.0 |
| Aluminium chlorohydrate (50 % soln.) | 40.0 |
| Sorbitan isostearate & polyglyceryl-3 ricinoleate | 1.0 |
| PPG-15 stearyl ether | 4.0 |
| Sorbeth-30 | 1.5 |
| Propylene glycol & diazolidinylurea & methyl paraben & propyl paraben | 0.25 |
| DI water | ad 100.0 |

Procedural - The emulsifier was dispersed in DI water and stirred until the starch was fully hydrated while heating to 75°C. The oil components were premixed, heated to 75°C and added to the water phase under homogenization for at least 5 minutes. Stirring continued while cooling; and the preservative was added at 30°C.

| | |
|---|---|
| Viscosity | 2,050 mPas |
| pH | 4.4 |

### EXAMPLE 6 - Salt Tolerance of Emulsions -

The following emulsions demonstrate the extraordinary salt tolerance of the emulsifier -

**Table 8 - Emulsifier with Salt**

| **INCI-Designation** | **6A** | **6B** | **6C** | **6D** |
|---|---|---|---|---|
| C₁₂₋₁₅ alkylbenzoate | 10.0 | 10.0 | 10.0 | 10.0 |
| Sample 2 | 5.0 | 5.0 | 5.0 | 5.0 |
| Phenoxyethanol & methyl paraben & ethyl paraben & propyl paraben | 1.0 | 1.0 | 1.0 | 1.0 |
| Sodium chloride | 2.0 | 10.0 | - | - |
| Calcium chloride | - | - | 2.0 | 10.0 |
| DI Water | ad 100.0 | ad 100.0 | ad 100.0 | ad 100.0 |

Procedural - The emulsifier was dispersed in DI water and stirred until hydration was fully achieved. The preservative, oil and salt were added while stirring at 900 rpm.

### EXAMPLE 7 - Compatibility with Oils -

The following emulsions demonstrate the broad compatibility with different oils as well as the excellent emulsifying properties being independent of HLB required by the oil phase -

**Table 9 - Emulsifier with Oil**

| **INCI-Designation** | **HLB** | **7A** | **7B** | **7C** | **7D** | **7E** | **7F** | **7G** | **7H** |
|---|---|---|---|---|---|---|---|---|---|
| Caprylic/capric triglyceride | 5 | 10.0 | | | | - | - | - | - |
| PPG-15 stearyl ether | 7 | - | 10.0 | | | - | - | - | - |
| Sunflower oil | 8 | - | - | 10.0 | | - | - | - | - |
| Cyclomethicone | 9 | - | - | - | 10.0 | - | - | - | - |
| Mineral oil | 10 | - | - | - | - | 10.0 | - | - | - |
| Isopropyl myristate | 11 | - | - | - | - | - | 10.0 | - | - |
| C₁₂₋₁₅ alkylbenzoate | 12 | - | - | - | - | - | - | 10.0 | - |
| C₁₂-C₁₄ Fatty Alcohol - | 14 | - | - | - | - | - | - | - | 10.0 |
| Sample 2 | | 5.0 | 5.0 | 5.0 | 1.0 | 5.0 | 5.0 | 5.0 | 5.0 |
| Phenoxyethanol & methyl dibromo glutaronitril | | 0.25 | 0.25 | 0.25 | 0.25 | 0.25 | 0.25 | 0.25 | 0.25 |
| DJ water | | q.s. | q.s. | q.s, | q.s. | q.s. | q.s. | q.s. | q.s. |
| Sum W/W% | | 100.0 | 100.0 | 100.0 | 100.0 | 100.0 | 100.0 | 100.0 | 100.0 |

Procedural - The emulsifier was dispersed in D.I. water and stirred until hydration was fully achieved. The preservative and oil were added while stirring using an overhead mixer at 900 rpm and homogenized for one minute at 13,000 rpm.

### EXAMPLE 8 - Compatibility with Rheology Modifiers and Bodying Systems

The following emulsions demonstrate the compatibility of the emulsifier with rheology modifiers and bodying systems -

**Table 10 - Emulsifier with Rheology Modifiers and Bodying Systems**

| **INCI-Designation** | **8A** | **8B** | **8C** | **8D** | **8E** |
|---|---|---|---|---|---|
| Cetyl alcohol | 3.0 | | | - | - |
| Stearic acid | - | 3.0 | | - | - |
| Cetearyl alcohol | - | - | 3.0 | - | - |
| Magnesium aluminium silicate | - | - | - | 0.8 | - |
| Xanthan gum | - | - | - | - | 0.2 |
| Mineral oil | 10.0 | 10.0 | 10.0 | 10.0 | 10.0 |
| Sample 2 | 5.0 | 5.0 | 1.0 | 5.0 | 5.0 |
| Phenoxyethanol & methyl dibromo glutaronitril | 0.25 | 0.25 | 0.25 | 0.25 | 0.25 |
| DI Water | ad 100.0 | ad 100.0 | ad 1.00.0 | ad 100.0 | ad 100.0 |

Procedural - The emulsifier was dispersed in DI water and stirred until hydration was fully achieved while heating to 75°C. The oils and rheology modifier were mixed together and heated to 75°C. The oil phase was added to the water phase while stirring. The preservative was added when the mixture was cooled to 40°C and then homogenized for one minute at 13,000 rpm.

## Claims

1. An emulsifier comprising:
a first starch component having at least one pregelatinized, crosslinked starch selected from a C₂-C₅ hydroxyalkyl starch and a C₂-C₁₈ acyl starch; and
a second starch component having at least one starch derivative, the starch derivative having a hydrophobic group or both a hydrophilic group and a hydrophobic group,
wherein the starch derivative is degraded by reaction with an exo-enzyme capable of cleaving 1,4- alpha -D-glucosidic linkages from non-reducing ends of starch but incapable of cleaving 1,6- alpha -D-glucosidic linkages of starch.

2. The emulsifier of claim 1, wherein the first starch component is crosslinked using phosphorylation or C₄-C₁₈ alkane or alkene dicarboxylic acids.

3. The emulsifier of claim 1, where the first starch component is crosslinked with a compound selected from the group consisting of phosphorous oxychloride, phosphorous pentoxide, sodium trimetaphosphate and mixtures thereof.

4. The emulsifier of claim 1, the first starch component comprising a pregelatinized, crosslinked, hydroxypropylated starch or a pregelatinized, crosslinked, acylated starch.

5. The emulsifier of claim 1, the first starch component comprising a pregelatinized hydroxypropyl di-starch phosphate.

6. The emulsifier of claim 1, the first starch component comprising a pregelatinized acetylated di-starch adipate.

7. The emulsifier of claim 1, wherein the second starch component is derivatized by reaction with an alkenyl cyclic dicarboxylic acid anhydride.

8. The emulsifier of claim 7, wherein the second starch component is derivatized using a compound selected from the group consisting of octenyl succinic acid anhydride, dodecenyl succinic acid anhydride and mixtures thereof.

9. The emulsifier of claim 1, wherein the second starch component is degraded using an enzyme selected from the group consisting of beta - amylase, exo- alpha -1,4-glucosidase, exo-1,4-alpha -D-glucan maltotetrahydrolase, and exo-1,4-alpha -D-glucan maltohexahydrolase.

10. The emulsifier of claim 1, the second starch component further comprising at least one starch derivative converted to a water fluidity of up to about 60.

11. The emulsifier of claim 1, wherein the first or second starch component is prepared from at least waxy corn starch.

12. The emulsifier of claim 1, wherein the first starch component and the second starch component are present in a ratio of 2:1.

13. A cosmetic or a personal care composition comprising the emulsifier of claim 1.

14. The emulsifier of claim 1 further comprising up to 10% salt.

15. A method of preparing a stable cosmetic or personal care composition comprising adding an effective amount of the emulsifier of claim 1.

## Patentansprüche

1. Ein Emulgator, umfassend:
eine erste Stärkekomponente mit mindestens einer vorgelatinierten, vernetzten Stärke, ausgewählt aus einer C₂-C₅-Hydroxyalkylstärke und einer C₂-C₁₈-Acyl-Stärke, und
eine zweite Stärkekomponente mit mindestens einem Stärkederivat, wobei das Stärkederivat einen hydrophoben Rest oder sowohl einen hydrophilen Rest als auch einen hydrophoben Rest aufweist,
wobei das Stärkederivat durch Reaktion mit einem Exo-Enzym abgebaut ist, das zur Spaltung von 1,4-alpha-D-glucosidischen Bindungen von nicht-reduzierenden Enden von Stärke fähig ist, aber unfähig zur Spaltung von 1,6-alpha-D-glucosidischen Bindungen von Stärke ist.

2. Der Emulgator nach Anspruch 1, wobei die erste Stärkekomponente mittels Phosphorylierung oder C₄-C₁₈-Alkan- oder Alken-Dicarbonsäuren vernetzt ist.

3. Der Emulgator nach Anspruch 1, wobei die erste Stärkekomponente mit einer Verbindung, ausgewählt aus der Gruppe bestehend aus Phosphoroxychlorid, Phosphorpentoxid, Natriumtrimetaphosphat und Gemischen davon vernetzt ist.

4. Der Emulgator nach Anspruch 1, wobei die erste Stärkekomponente eine vorgelatinierte vernetzte, hydroxypropylierte Stärke oder eine vorgelatinisierte, vernetzte, acylierte Stärke umfasst.

5. Der Emulgator nach Anspruch 1, wobei die erste Stärkekomponente ein vorgelatiniertes Hydroxypropyl-Di-Stärkephosphat umfasst.

6. Der Emulgator nach Anspruch 1, wobei die erste Stärkekomponente einen vorgelatinierten acetylierten Di-Stärkeadipatester umfasst.

7. Der Emulgator nach Anspruch 1, wobei die zweite Stärkekomponente durch Umsetzung mit einem cyclischen Alkenyl-Dicarbonsäureanhydrid derivatisiert ist.

8. Der Emulgator nach Anspruch 7, wobei die zweite Stärkekomponente unter Verwendung einer Verbindung, ausgewählt aus der Gruppe bestehend aus Octenylbernsteinsäureanhydrid, Dodecenylbemsteinsäureanhydrid und Gemischen davon derivatisiert ist.

9. Der Emulgator nach Anspruch 1, wobei die zweite Stärkekomponente unter Verwendung eines Enzyms, ausgewählt aus der Gruppe bestehend aus beta-Amylase, exo-alpha-1,4-Glucosidase, exo-1,4-alpha-D-Glucan-Maltotetrahydrolase und exo-1,4-alpha-D-Glucan-Maltohexahydrolase abgebaut ist.

10. Der Emulgator nach Anspruch 1, wobei die zweite Stärkekomponente darüber hinaus mindestens ein Stärkederivat umfasst, das zu einer Wasserfließfähigkeit von bis zu etwa 60 umgewandelt ist.

11. Der Emulgator nach Anspruch 1, wobei die erste oder zweite Stärkekomponente aus mindestens Wachsmaisstärke hergestellt ist.

12. Der Emulgator nach Anspruch 1, wobei die erste Stärkekomponente und die zweite Stärkekomponente in einem Verhältnis von 2:1 vorhanden sind.

13. Eine kosmetische oder Körperpflege-Zusammensetzung, die den Emulgator nach Anspruch 1 umfasst.

14. Der Emulgator nach Anspruch 1, der darüber hinaus bis zu 10% Salz umfasst.

15. Verfahren zur Herstellung einer stabilen kosmetischen oder Körperpflege-Zusammensetzung, umfassend das Zugeben einer wirksamen Menge des Emulgators nach Anspruch 1.

## Revendications

1. Émulsifiant comprenant:
un premier composant d'amidon ayant au moins de l'amidon prégélatinisé, réticulé choisi parmi de l'amidon hydroxyalkyle en C₂ à C₅ et de l'amidon acyle en C₂ à C₁₈ et
un deuxième composant d'amidon ayant au moins un dérivé d'amidon, le dérivé d'amidon ayant un groupe hydrophobe ou à la fois un groupe hydrophile et un groupe hydrophobe,
dans lequel le dérivé d'amidon est dégradé par réaction avec une exo-enzyme capable de cliver des liaisons 1,4-alpha-D-glucosidiques à partir des extrémités non-réductrices de l'amidon mais incapable de cliver des liaisons 1,6-alpha-D-glucosidiques de l'amidon.

2. Émulsifiant de la revendication 1, dans lequel le premier composant d'amidon est réticulé en utilisant la phosphorylation ou des acides alcane ou alcène en C₄ à C₁₈ dicarboxyliques.

3. Émulsifiant de la revendication 1, dans lequel le premier composant d'amidon est réticulé avec un composé choisi dans le groupe constitué d'oxychlorure de phosphore, de pentoxyde de phosphore, de trimétaphosphate de sodium et des mélanges de ceux-ci.

4. Émulsifiant de la revendication 1, le premier composant d'amidon comprenant de l'amidon prégélatinisé, réticulé, hydroxypropylé ou de l'amidon prégélatinisé, réticulé, acylé.

5. Émulsifiant de la revendication 1, le premier composant d'amidon comprenant du phosphate de diamidon hydroxypropylique prégélatinisé.

6. Émulsifiant de la revendication 1, le premier composant d'amidon comprenant un adipate de diamidon acétylé prégélatinisé.

7. Émulsifiant de la revendication 1, dans lequel le deuxième composant d'amidon est dérivé par réaction avec un anhydride d'acide alcényldicarboxylique cyclique.

8. Émulsifiant de la revendication 7, dans lequel le deuxième composant d'amidon est dérivé en utilisant un composé choisi dans le groupe constitué d'anhydride d'acide octénylsuccinique, d'anhydride d'acide dodécénylsuccinique et des mélanges de ceux-ci.

9. Émulsifiant de la revendication 1, dans lequel le deuxième composant d'amidon est dégradé en utilisant une enzyme choisie dans le groupe constitué de bêta-amylase, d'exo-alpha-1,4-glucosidase, d'exo-1,4-alpha-D-glucane maltotétrahydrolase, et d'exo-1,4-alpha-D-glucane maltohexahydrolase.

10. Émulsifiant de la revendication 1, le deuxième composant d'amidon comprenant en outre au moins un dérivé d'amidon converti à une fluidité dans l'eau allant jusqu'à environ 60.

11. Émulsifiant de la revendication 1, dans lequel le premier ou le deuxième composant d'amidon est préparé à partir d'au moins de l'amidon de maïs cireux.

12. Émulsifiant de la revendication 1, dans lequel le premier composant d'amidon et le deuxième composant d'amidon sont présents en un rapport de 2:1.

13. Composition cosmétique ou de soins personnels comprenant l'émulsifiant de la revendication 1.

14. Émulsifiant de la revendication 1, comprenant en outre jusqu'à 10% de sel.

15. Procédé de préparation d'une composition cosmétique ou de soins personnels stable comprenant l'ajout d'une quantité efficace de l'émulsifiant de la revendication 1.
